# EUROPEAN PATENT SPECIFICATION

(11) **EP 4 296 652 B1**
(45) Date of publication and mention of the grant of the patent: **28.10.2026**
(21) Application number: 23179541.0
(22) Date of filing: 15.06.2023
(51) Int. Cl.: G01N 21/39, G01N 33/00, G01N 33/22, G01N 21/35, G01N 21/03, G01N 21/3504, G01N 21/31

(54) **ENHANCEMENTS TO LASER SPECTROSCOPY MODELING BY MEASUREMENT OF HYDROCARBON FUEL GAS COMPOSITIONS**
VERBESSERUNGEN AN LASERSPEKTROSKOPIEMODELLIERUNG DURCH MESSUNG VON KOHLENWASSERSTOFF-BRENNGASZUSAMMENSETZUNGEN
AMÉLIORATIONS APPORTÉES À LA MODÉLISATION PAR SPECTROSCOPIE LASER PAR MESURE DE COMPOSITIONS DE GAZ COMBUSTIBLE HYDROCARBONÉ

(30) Priority: 24.06.2022 US 202217849360
(43) Date of publication of application: 27.12.2023
(73) Proprietor: ABB Schweiz AG, 5400 Baden (CH)
(72) Inventor: DESBIENS, Raphaël, Quebec, G1C8C2 (CA); LEEN, John Brian, California, 94087 (US); OWEN, Kyle, California, 95134 (US)
(74) Representative: Zimmermann & Partner Patentanwälte mbB

(56) References cited:
- US-A- 6 115 673
- US-A1- 2017 191 929
- US-A1- 2018 095 031

## Description

The present disclosure relates to measuring chemical constituents and associated properties of hydrocarbon fuel mixtures, and further relates to tunable diode laser absorption spectrometry gas analyzers having improved chemometric models.

Whenever fuel gas (natural gas, coal syngas, biogas, etc.) is generated, transferred or used, knowledge of the fuel gas' level of contamination, heating value, relative density, compressibility, theoretical hydrocarbon liquid content, and Wobbe index are typically required. Measurement of various target components, such as gas contaminants (e.g. H₂S, H₂O, O₂, CO₂) is critical for preventing infrastructure damage due to corrosion or chemical reactivity. Natural gas producers must clean extracted natural gas to remove contaminants and then verify any residual contaminant levels before the fuel gas is introduced into a pipeline. Desulfurizer beds in fuel reformers need periodic replacement or regeneration to prevent H₂S breakthrough into the reformed fuel product, and so require frequent contaminant level monitoring. Accurate measurement and monitoring of key gas parameters, including heating value, relative density, compressibility, theoretical hydrocarbon liquid content, and Wobbe index, are critical for pricing the fuel, optimizing burner conditions, and determining combustion efficiency.

Laser absorption spectrometers draw a sample of fuel gas from a gas stream to measure the total light absorption spectrum associated with the gas sample. The laser absorption spectrometer is calibrated to measure light absorption of the various contaminants within the gas sample, however, background gases (such as non-contaminants methane and propane, or other dominant constituents of natural gas, coal syngas and biogas), can affect the light absorption measurement of the contaminants. Empirical models, multivariate fitting routines, or more generally post-processing of the measured light absorption of the contaminants are used to determine the amount of the various contaminants present in the gas mixture. A multitude of input parameters and iterative calibration processes are implemented within the post-processing methods to yield an empirical approximation of the various contaminants present in the gas mixture. Such approximations are accurate where the input parameters are known, however approximating the contaminants is difficult because the gas concentration of background gasses are often variable, leading to inaccuracies in the post-processing and tedious post-calibration procedures. US 6 115 673 A describes an apparatus and a method for generating basis sets for use in spectroscopic analysis. US 2017/191929 A1 describes a method for spectral interpretation in absorption spectroscopy uses a nonlinear spectral fitting algorithm for interpretation of spectral features in complex absorption spectra. US 2018/095031 A1 describes the measuring of chemical constituents and associated properties of hydrocarbon fuel mixtures.

Therefore, there exists a need in the art to improve systems and methods of measuring gas contaminants using laser absorption spectrometry.

### BRIEF DESCRIPTION

In one aspect, method for determining target components in a sample gas mixture is described. The method includes obtaining measured gas concentration data for each of one or more background gases present in the sample gas mixture from a concentration measurement instrument, the gas concentration measurement instrument configured to measure gas concentrations of the one or more background gases; obtaining measured light absorption data of each of one or more gaseous target components from a laser spectroscopy instrument indicative of an amount of absorption of light by the sample gas mixture at a frequency of each of the one or more gaseous target components, the laser spectroscopy instrument configured to measure light absorption of the one or more gaseous target components within the sample gas, each of the one or more gaseous target components having an absorption spectrum at the frequency of light or at a combination of frequencies of frequencies of light; setting gas concentration fit coefficients to a chemometric model for the measured gas concentration data for each of one or more background; wherein the chemometric model employs a broadband offset basis to a final basis set spectrum for the one or more gaseous target components; applying an iterative mathematical model to the measured light absorption data by iteratively adjusting target component fit coefficients until the measured light absorption data matches the chemometric model; and, determining a calculated amount of the one or more target components within the sample gas mixture by applying the gas concentration fit coefficients and the target component fit coefficients to the measured light absorption data and to the measured gas concentration data.

In another aspect, a method for determining target components in a sample gas mixture is described. The method includes obtaining measured gas concentration data for each of one or more background gases present in the sample gas mixture from a concentration measurement instrument, the gas concentration measurement instrument configured to measure gas concentrations of the one or more background gases; obtaining measured light absorption data of each of one or more gaseous target components from a laser spectroscopy instrument indicative of an amount of absorption of light by the sample gas mixture at a frequency of each of the one or more gaseous target components, the laser spectroscopy instrument configured to measure light absorption of the one or more gaseous target components within the sample gas, each of the one or more gaseous target components having an absorption spectrum at the frequency of light or at a combination of frequencies of frequencies of light; applying an iterative mathematical model to the measured light absorption data by iteratively adjusting gas concentration fit coefficients and target component fit coefficients until the measured light absorption data matches the chemometric model; wherein the chemometric model employs a broadband offset basis to a final basis set spectrum for the one or more gaseous target components; applying a correction function to the iterative mathematical model, wherein input parameters of the correction function are the measured gas concentration data and one or more of the iterated gas concentration fit coefficients and target component fit coefficients from the iterative mathematical model; and, determining a calculated amount of the one or more target components within the sample gas mixture by applying the fit coefficient to the measured light absorption data and to the measured gas concentration data

### BRIEF DESCRIPTION OF THE DRAWINGS

FIG. 1 is a schematic perspective view of an off-axis ICOS instrument in accord with the present invention;
FIG. 2 is a graph of cavity-enhanced absorption versus optical frequency for representative basis sets for analysis of H2S and CO2 in natural gas;
FIG. 3 is a schematic representation of a light laser absorption system;
FIG. 4 illustrates a method for determining target components in the fuel gas stream pre-fit.
FIG. 5 illustrates a method for determining target components in the fuel gas stream post-fit.

The reference symbols used in the drawings, and their meanings, are listed in summary form in the list of reference symbols. In principle, identical parts are provided with the same reference symbols in the figures.

### DETAILED DESCRIPTION

In the following specification and the claims, reference will be made to a number of terms, which shall be defined to have the following meanings.

As used herein, the singular forms "a," "an," and "the" include plural references unless the context clearly dictates otherwise. The terms "comprising," "including," and "having" are intended to be inclusive and mean that there may be additional elements other than the listed elements. The terms "optional" or "optionally" means that the subsequently described event or circumstance may or may not occur, and that the description includes instances where the event occurs and instances where it does not.

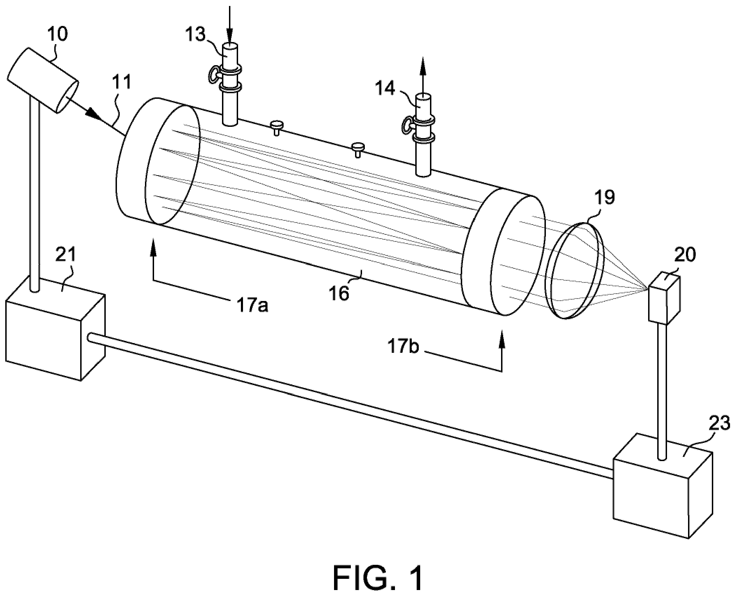

With reference to FIG. 1, an off-axis ICOS instrument 10 like that described in U.S. Pat. No. 6,795,190 is shown. Laser light 11 from a tunable near-infrared diode laser 10 is coupled off-axis into a high-finesse optical cavity 16 with two highly reflective (R~99.995%) mirrors 17a and 17b, while fuel gas to be analyzed is flowed through the cavity 16 between gas inlet 13 and gas outlet 14. A near-infrared sensor 20 measures the intensity of light exiting the cavity via a lens 19 as the laser wavelength is tuned over a specified range by means of laser control electronics 21, thereby providing a transmission spectrum that measures wavelength-dependent optical absorption by all of the various chemical components present in the fuel gas. The illustrated embodiment utilizes Off-Axis ICOS; however, other tunable diode laser absorption spectrometry instruments and methods may also be used.

Choice of wavelength range of the lasers depends upon the chemical species to be detected, avoiding where possible interfering absorptions from different species. Multiple laser diodes may be available for providing absorption measurements over several different ranges. Some embodiments utilize two lasers; for example, laser 10 operating near 1.58 µm and 1.27 µm, but other choices are possible. The spectral range over which each laser diode may be tuned is at least 20 GHz and preferably 60-80 GHz. Sensor data is collected and analyzed by a computer system 23, which in accord with the present disclosure employs chemometric fitting routines and calculations of heating value, relative density, compressibility, theoretical hydrocarbon liquid content, Wobbe index, and contaminant concentrations for the fuel gas stream.

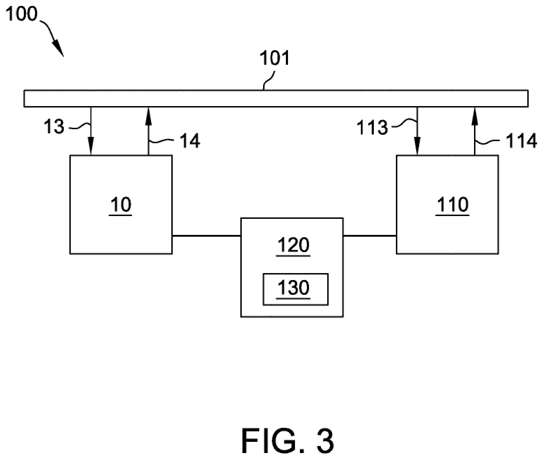

FIG. 3 illustrates a schematic representation of a light laser absorption system 100 including the off-axis ICOS instrument 10 having the gas inlet 13 and gas outlet 14 connected to a fuel gas stream 101, and a gas concentration measurement instrument 110 having a gas inlet 113 and gas outlet 114 connected to the gas stream 101. The off-axis ICOS instrument 10 and the gas concentration measurement instrument 110 draw samples from the same gas stream 101. In some embodiments, the off-axis ICOS instrument 10 and the gas concentration measurement instrument 110 are connected in series and draw the same gas sample from the gas stream 101. The off-axis ICOS instrument 10 and the gas concentration measurement instrument 110 are communicatively connected to a processor 120 and memory 130, where the processor is configured to perform operations (200, 300, 400) as described herein, and the memory 130 is configured to store at least developed tables and light absorption spectra profiles, as well as the stored basis sets for use with the chemometric modeling, including individual spectra from each of the expected target components in the fuel gas for the wavelength ranges being scanned by the off-axis ICOS instrument 10 (or more generally a laser spectroscopy device)

The processor 120 and memory 130 are configured to perform mathematical algorithms, mathematical modeling, iterative modeling, regression modeling, linear regression modeling, line fitting and the like using commercially available or proprietary software or such as MATLAB by MathWorks ^{©}. The memory 130 is configured to store such software, as well as chemometric models and final basis set spectra as explained in further detail below. The processor 120 is configured to receive data from the off-axis ICOS instrument 10 (or more generally a laser spectroscopy instrument) and the gas concentration measurement instrument 110 and input said data into the chemometric models, which can provide as output modeled values for said measured data.

In some embodiments, the off-axis ICOS instrument 10 is a laser spectroscopy device. In some embodiments, the gas concentration measurement instrument 110 is selected from a group consisting of a gas chromatography instrument, a Fourier-transform infrared spectroscopy instrument, a gas concentration sensor, a metal oxide gas sensor, an electrochemical gas sensor, a dynamic zirconia dioxide sensor and a catalytic gas sensor. The gas concentration measurement instrument 110 is configured to measure or determine gas concentrations of background gasses (such as non-contaminants methane and propane, or more generally other dominant constituents of natural gas, coal syngas and biogas).

As used herein, the term "chemometric model" shall mean a mathematical model stored in memory 130 and executed by the processor 120. The chemometric model employs a broadband offset basis to a final basis set spectrum for the one or more gaseous target components, which include spectra of target components (i.e. contaminants) found in gas streams and the background (base or constituent) components of the gas streams. The spectra (modeled wavelengths) can be modeled for variations in concentrations of any of the target components or background components, and a multitude of static parameters, variable parameters, gain factors and fit coefficients may be applied to the chemometric model such that the processor 120 yields a resultant measurement.

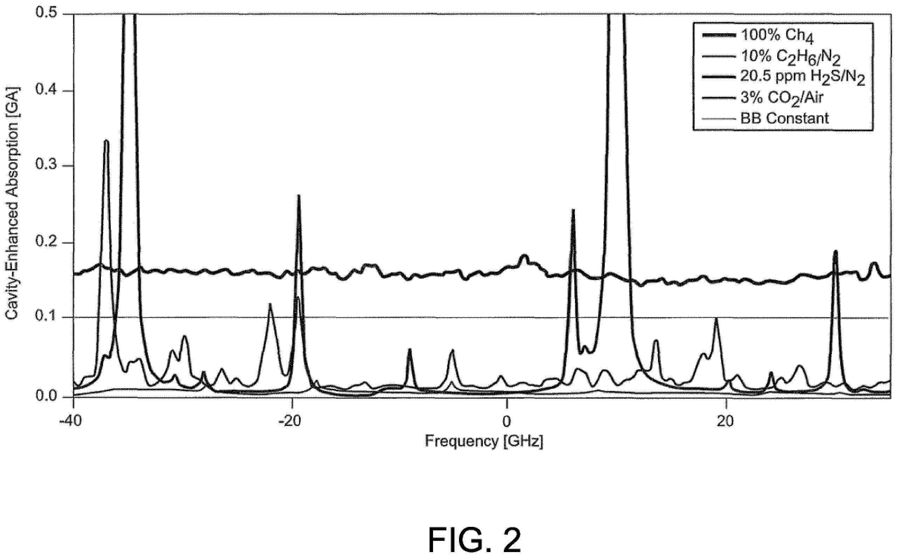

FIG. 2 shows a representative basis set for chemometric fitting and analysis of natural gas with possible contaminants H₂S and CO₂ for the vicinity (-40 GHz to +30 GHz) of 1.58 µm, where the background gas concentrations are fixed or otherwise certified. In the illustrated figure, the basis set includes methane, ethane, and broadband constant that represents higher hydrocarbons and other relatively featureless absorptions. From this graph, the cavity-enhanced absorption (the y-axis) equals the cavity gain factor G (=R/(1-R), where R is the mirror reflectivity) multiplied by the single-pass absorption A.

To facilitate line fitting mathematical processes using software (hereinafter reffered to as "line fitting") of the measured spectrum, the stored basis sets for use with the chemometric modeling can be stored in memory 130 (as shown in FIG. 3) and should include individual spectra from each of the expected components in the fuel gas for the wavelength ranges being scanned by the instrument. By way of example, an absorption spectrum of pure methane (CH₄) (the dominant constituent of natural gas, coal syngas and biogas) is stored in memory 130. In some embodiments, absorption spectra for each of the dominant constituents are included in memory 130. The terms, "base gases" and "dominant" gases or constituents are hereinafter referred to "background gases" and they are the gaseous chemicals which comprise natural gas, or more generally gases transferred through gas streams.

The methane (CH₄) spectrum (or any of the background gases) is included in a basis set spectra as shown in FIG. 2 and is typically highly structured. Likewise, the basis set spectra also includes the absorption spectrum of ethane (C₂H₆). Because the expected percentage of ethane (C₂H₆) in the fuel gas mixture is lower than other background gases, it is convenient that the basis set spectrum employed be that of a mixture of 10% ethane in inert nitrogen background. The absorption spectra of target components (e.g. H₂S, H₂O, O₂, and CO₂) measured in an inert background (e.g. nitrogen or zero-air) are also included. These spectra are typically highly structured and can be measured by the laser spectroscopy instrument, producing a spectra as shown in FIG. 2. The system is not limited to any particular set of fuel gas components and contaminants and can be extended to other gases with or without fuel values (H₂, OCS, etc.) provided a basis spectrum is available for use in the fitting operation. All of these absorption spectra may be empirically determined by filling the cavity with certified concentrations of the components diluted in dry air, nitrogen or other inert gas, and taking the spectra under similar conditions (temperature, pressure, etc.) as the fuel gas measurements to be made. The final basis set "spectrum" included in memory 130 with the chemometric model is a broadband offset basis that is totally featureless (e.g. 10% absorption at all measured wavelengths). This accounts for essentially featureless or otherwise constant broadband absorptions by all higher hydrocarbons over the selected wavelength ranges. As used herein, the term "structured" when reffering to background gases or target components shall denote a gaseous compound which produces a spectrum for a given sensitivity of the laser spectroscopy instrument. By way of example, the laser spectroscopy instrument may only be tuned and calibrated for some gaseous compounds but not others.

Thus, where known or certified concentrations of background gases are properly modeled, the final basis set spectrum can be modeled by the broadband offset basis as previously described, and included in the basis set spectra (as shown in FIG. 2). However, where variable concentrations of background gases are present, the model must be adjusted as such by adjusting one or more fit coefficients of the model. Stated differently, the offset of the final basis set spectrum of the measurement can be empirically calculated using modeling implemented by software as previously described when the background gas concentrations is certified, but variations in background gas concentrations increase model complexity, computation time and can introduce error into the model for measuring wavelengths without proper adjustment of the broadband offset (by way of adjusting or setting one or more fit coefficients of the model). The chemometric model includes a multitude of fit coefficients which can correspond to parameters such as the concentrations of background gases, concentrations of target components, measured light absorption of background gases and measured light absorption of target components. Where a parameter is known, the fit coefficient can be inputted into the model by either the off-axis ICOS instrument 10 (or more generally a laser spectroscopy instrument), the gas concentration measurement instrument 110, or by a user. In some embodiments, where a given parameter does not result in appreciable error of the model, the parameter can be omitted entirely to reduce complexity of the model and computational time. In some embodiments, unknown parameters can be approximated using iterative modeling, regression modeling or other mathematical algorithms known in the art. By way of example, an iterative modeling can be applied, and is reffered to herein as an "iterative model."

A chemometric data analysis strategy like that described in Linh D. Le et al., "Development of a Rapid On-Line Acetylene Sensor for Industrial Hydrogenation Reactor Optimization Using Off-Axis Integrated Cavity Output Spectroscopy", Applied Spectroscopy 62(1), pp. 59-62 (2008) is one known way to quantify the respective constituents. Background, non-contaminant gases are strong broadband absorbers and measurement of the contaminant gasses can be influenced by the background gas leading to inaccurate results. For example, a measurement of target component H₂S at 0, 10, or 20 ppm in a background gases of CH₄ and C₃H₈ will produce varied measurements. This is due to collisional broadening from the background gases present in the gaseous mixture. The broadening is different because natural gas has different gases than in the certified inert nitrogen background the model is based on. Generally, broadening denotes enlargement of the recorded spectrum stored in memory 130.

Mixture concentrations of the background gases must therefore be incorporated into the empirical model. Even if the background gases are properly fit by iterative approximation; if the broadening is not correctly accounted for the model will not match the measurement accurately. Fitting algorithms can be implemented to minimize measurement errors from interfering absorptions of background gases. The fit algorithm also includes a fit of the baseline to compensate for any low-frequency perturbation, like a change in the laser intensity or change in the cell transmission. Knowing the actual broadband absorber composition allows for the inclusion of the theoretical absorption line in the model or adjust the calculated concentration value to correct for the change in background gas. One or more fit coefficients for the concentrations or light absorption of background gases or target components can therefore be applied to the chemometric model.

Embodiments of the present disclosure utilize instrumentation (such as the gas concentration measurement instrument 110 of FIG. 3) to measure gas concentrations of background gases and incorporate the measured gas concentrations into the model to correct the measurement of gas contaminants by applying a fit coefficient of the gas concentration of background gases or target components to the model either post-fit or pre-fit for the chemometric fitting routines. In some embodiments, the model can be adjusted to account for the concentration of the background gasses using fitting algorithms and/or iterative formulas. In some embodiments, the concentration of the background gasses can be measured and inputted into the model and subsequently correcting the output using a correction function or look up table stored in memory 130. In some embodiments, the background gasses can be inputted by a user. In some embodiments, where a particular background gas does not result in appreciable error of the model, the concentration of the background gas can be omitted entirely.

As previously described, the off-axis ICOS instrument 10 employs chemometric fitting routines and empirically contaminant concentrations for the fuel gas stream 101 from the final basis set spectrum included in memory 130. The chemometric model employs a broadband offset basis to the final basis set spectrum for measurement of target components (e.g. H₂S, H₂O, O₂, and CO₂) as described in U.S. Pat. No. 6,795,190. Thus, where known or certified concentrations of background gases are properly modeled, the final basis set spectrum can be determined by modeling or by tabulation tables. However, where variable concentrations of background gases are present, the model must be adjusted by the fit coefficients, or the concentrations must be measured and fit into the model. Stated differently, the offset of the final basis set spectrum of the measurement can be empirically calculated as previously described when the background gas concentrations are known or by using certified gas concentrations, but variations in background gas concentrations increase model complexity and can introduce error into the model for measuring wavelengths without proper adjustment of the model and in particular the broadband offset basis of the model. By determining the gas concentrations of background gasses in a mixture from the gas concentration measurement instrument 110, a mathematical algorithm and theoretical transformation can be implemented to properly fit the model.

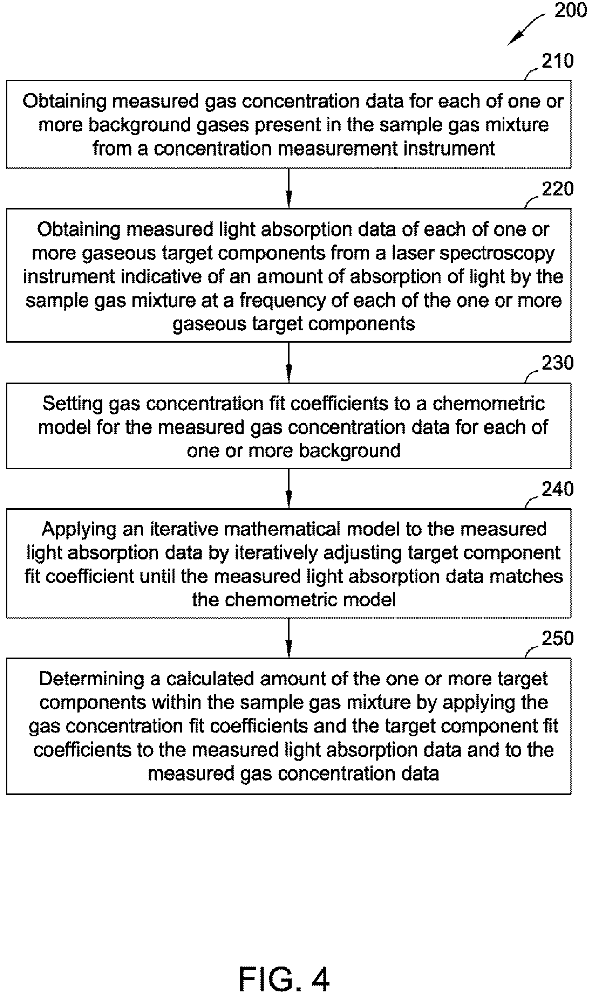

As shown in FIG. 4, in some embodiments, a method 200 for determining target components in the fuel gas stream 101 pre-fit is described. The method 200 can be performed as a computer-implemented method where the by processor 120 of FIG. 3 instructs the gas concentration measurement instrument 110 and the off-axis ICOS instrument 10 to perform the method 200. The off-axis ICOS instrument 10, or more generally, a laser absorption instrument is configured to measure light absorption of one or more gaseous contaminants within a sample gas mixture of one or more background gases, with each of the one or more target components having an absorption spectrum at a frequency of light or at a combination of frequencies of light. The gas concentration measurement instrument 110 is configured to measure gas concentrations of the one or more background gases.

The method 200 includes obtaining 210 measured gas concentration data for each of the one or more background gases present in the fuel gas stream 101 from the concentration measurement instrument 110. In some embodiments, the measured gas concentration data is stored in the memory 130 of FIG. 3. The method 200 further includes obtaining 220 measured light absorption data of each of one or more gaseous target components from a laser spectroscopy instrument indicative of an amount of absorption of light by the sample gas mixture at a frequency of each of the one or more gaseous target components, the laser spectroscopy instrument configured to measure light absorption of the one or more gaseous target components within the sample gas, each of the one or more gaseous target components having an absorption spectrum at the frequency of light or at a combination of frequencies of frequencies of light. In some embodiments, the method 200 further includes setting 230 gas concentration fit coefficients to a chemometric model for the measured gas concentration data for each of one or more background; wherein the chemometric model employs a broadband offset basis to a final basis set spectrum for the one or more gaseous target components, and applying 240 an iterative mathematical model to the measured light absorption data by iteratively adjusting target component fit coefficients until the measured light absorption data matches the chemometric model.

The method 200 further includes determining 250 a calculated amount of the one or more target components within the sample gas mixture by applying the gas concentration fit coefficients and the target component fit coefficients to the measured light absorption data and to the measured gas concentration data.

In some embodiments, the processor 120 of FIG. 3 is configured to compare the measured light absorption data of each of one or more gaseous target components against the chemometric model; compare the measured gas concentration data for each of one or more background gases against a model of certified concentrations of background gases; apply an iterative mathematical model to the measured light absorption data by iteratively adjusting a fit coefficient until the measured light absorption data matches the chemometric model; and, determine a calculated amount of the one or more target components within the sample gas mixture by applying the fit coefficient to the measured light absorption data and to the measured gas concentration data. In some embodiments, the measurement absorption data of each of the one or more target components is adjusted by instructing the processor to apply an iterative correction function to the measured absorption data of each of the one or more target components against a model light absorption of the one or more target components in the standard gas concentration of the one or more background gases.

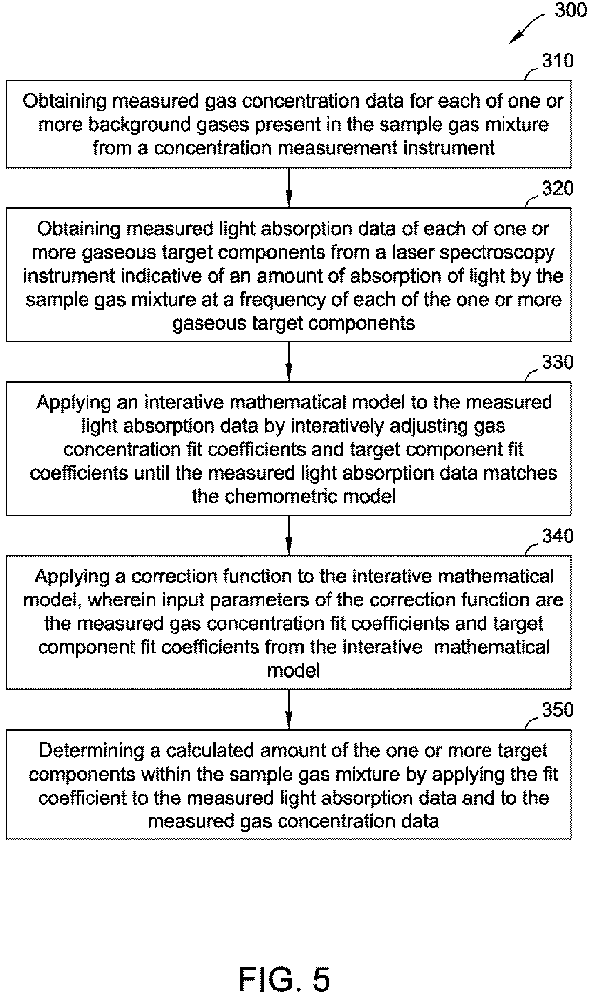

As shown in FIG. 5, in some embodiments, a method 300 for determining target components in the fuel gas stream 101 post-fit is described. The method 300 can be performed as a computer-implemented method where the by processor 120 of FIG. 3 instructs the gas concentration measurement instrument 110 and the off-axis ICOS instrument 10 to perform the method 300. The off-axis ICOS instrument 10, or more generally, a laser absorption instrument is configured to measure light absorption of one or more gaseous contaminants within a sample gas mixture of one or more background gases, with each of the one or more target components having an absorption spectrum at a frequency of light or at a combination of frequencies of light. The gas concentration measurement instrument 110 is configured to measure gas concentrations of the one or more background gases.

The method 300 includes obtaining 310 measured gas concentration data for each of the one or more background gases present in the fuel gas stream 101 from the concentration measurement instrument 110. In some embodiments, the measured gas concentration data is stored in the memory 130 of FIG. 3. The method 300 further includes obtaining 320 measured light absorption data of each of one or more gaseous target components from a laser spectroscopy instrument indicative of an amount of absorption of light by the sample gas mixture at a frequency of each of the one or more gaseous target components, the laser spectroscopy instrument configured to measure light absorption of the one or more gaseous target components within the sample gas, each of the one or more gaseous target components having an absorption spectrum at the frequency of light or at a combination of frequencies of frequencies of light.

In some embodiments, the method 300 further includes applying 330 an iterative mathematical model to the measured light absorption data by iteratively adjusting gas concentration fit coefficients and target component fit coefficients until the measured light absorption data matches the chemometric model; wherein the chemometric model employs a broadband offset basis to a final basis set spectrum for the one or more gaseous target components.

The method 300 further includes applying 340 a correction function to the iterative mathematical model, wherein input parameters of the correction function are the measured gas concentration data and one or more of the iterated gas concentration fit coefficients and target component fit coefficients from the iterative mathematical model. The method 300 finally includes determining 350 a calculated amount of the one or more target components within the sample gas mixture by applying the fit coefficient to the measured light absorption data and to the measured gas concentration data.

In some embodiments, applying the correction function includes comparing the measured gas concentration data against a look up table stored in memory

Other variations to the disclosed embodiments can be understood and effected by those skilled in the art in practicing the claimed disclosure, from the study of the drawings, the disclosure, and the appended claims. In the claims the word "comprising" does not exclude other elements or steps and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope of the claims.

While the disclosure has been illustrated and described in detail in the drawings and foregoing description, such illustration and description are to be considered illustrative or exemplary and not restrictive. It will be understood that changes and modifications may be made by those of ordinary skill within the scope of the following claims.

The use of the article "a" or "the" in introducing an element should not be interpreted as being exclusive of a plurality of elements. Likewise, the recitation of "or" should be interpreted as being inclusive, such that the recitation of "A or B" is not exclusive of "A and B," unless it is clear from the context or the foregoing description that only one of A and B is intended. Further, the recitation of "at least one of A, B and C" should be interpreted as one or more of a group of elements consisting of A, B and C, and should not be interpreted as requiring at least one of each of the listed elements A, B and C, regardless of whether A, B and C are related as categories or otherwise. Moreover, the recitation of "A, B and/or C" or "at least one of A, B or C" should be interpreted as including any singular entity from the listed elements, e.g., A, any subset from the listed elements, e.g., A and B, or the entire list of elements A, B and C.

This written description uses examples to disclose the disclosure, including the best mode, and also to enable any person skilled in the art to practice the disclosure, including making and using any devices or systems and performing any incorporated methods. The patentable scope of the disclosure is defined by the claims, and may include other examples that occur to those skilled in the art within the scope of the claims if they have structural elements that do not differ from the literal language of the claims, or if they include equivalent structural elements with insubstantial differences from the literal languages of the claims.

## Claims

1. A method (200) for determining target components in a sample gas mixture comprising:
obtaining (210) measured gas concentration data for each of one or more background gases present in the sample gas mixture from a concentration measurement instrument (110), the gas concentration measurement instrument configured to measure gas concentrations of the one or more background gases;
obtaining (220) measured light absorption data of each of one or more gaseous target components from a laser spectroscopy instrument indicative of an amount of absorption of light by the sample gas mixture at a frequency of each of the one or more gaseous target components, the laser spectroscopy instrument configured to measure light absorption of the one or more gaseous target components within the sample gas, each of the one or more gaseous target components having an absorption spectrum at the frequency of light or at a combination of frequencies of frequencies of light;
setting (230) gas concentration fit coefficients to a chemometric model for the measured gas concentration data obtained for each of the one or more background gases present in the sample gas mixture, wherein the chemometric model employs a broadband offset basis, accounting for absorptions by the one or more background gases, in a final basis set spectrum for the one or more gaseous target components;
applying (240) an iterative mathematical model to the measured light absorption data by iteratively adjusting target component fit coefficients until the measured light absorption data matches the chemometric model; and
determining (250) a calculated amount of the one or more target components within the sample gas mixture by applying the gas concentration fit coefficients and the target component fit coefficients to the measured light absorption data and to the measured gas concentration data of the one or more background gases.

2. The method of claim 1, wherein the iterative mathematical model is a regression model.

3. The method of claim 1 or 2, wherein a processor (120) is communicatively connected to the laser spectroscopy instrument and the gas concentration measurement instrument, the processor instructed to:
set gas concentration fit coefficients to the chemometric model for the measured gas concentration data for each of one or more background;
apply the iterative mathematical model to the measured light absorption data by iteratively adjusting target component fit coefficients until the measured light absorption data matches the chemometric model; and
determine the calculated amount of the one or more target components within the sample gas mixture by applying the gas concentration fit coefficients and the target component fit coefficients to the measured light absorption data and to the measured gas concentration data.

4. The method of claim 3, wherein the measurement absorption data of each of the one or more target components is adjusted by instructing the processor to apply an iterative correction function to the measured absorption data of each of the one or more target components against a model light absorption of the one or more target components in the standard gas concentration of the one or more background gases.

5. The method of any one of the preceding claims, wherein the laser spectroscopy instrument is an off-axis integrated cavity output spectroscopy device (10).

6. The method of any one of the preceding claims, wherein the gas concentration measurement instrument is selected from a group consisting of a gas chromatograph, a Fourier-transform infrared spectroscopy device, a gas concentration sensor, a metal oxide gas sensor, an electrochemical gas sensor, a dynamic zirconia dioxide sensor and a catalytic gas sensor.

7. The method of any one of the preceding claims, wherein the one or more gaseous target components are gaseous contaminants found in natural gas.

8. The method of any one of the preceding claims, wherein the gaseous contaminants are one or more of H₂S, H₂O, O₂, or CO₂.

9. A method (300) for determining target components in a sample gas mixture comprising:
obtaining (310) measured gas concentration data for each of one or more background gases present in the sample gas mixture from a concentration measurement instrument (110), the gas concentration measurement instrument configured to measure gas concentrations of the one or more background gases;
obtaining (320) measured light absorption data of each of one or more gaseous target components from a laser spectroscopy instrument indicative of an amount of absorption of light by the sample gas mixture at a frequency of each of the one or more gaseous target components, the laser spectroscopy instrument configured to measure light absorption of the one or more gaseous target components within the sample gas, each of the one or more gaseous target components having an absorption spectrum at the frequency of light or at a combination of frequencies of frequencies of light;
applying (330) an iterative mathematical model to the measured light absorption data by iteratively adjusting gas concentration fit coefficients and target component fit coefficients until the measured light absorption data matches the chemometric model, wherein the chemometric model employs a broadband offset basis, accounting for absorptions by the one or more background gases, in a final basis set spectrum for the one or more gaseous target components;
applying (340) a correction function to the iterative mathematical model, wherein input parameters of the correction function are the measured gas concentration data and one or more of the iterated gas concentration fit coefficients and target component fit coefficients from the iterative mathematical model; and
determining (350) a calculated amount of the one or more target components within the sample gas mixture by applying the iterated fit coefficients to the measured light absorption data and to the measured gas concentration data of the one or more background gases.

10. The method of claim 9, wherein applying the correction function includes comparing the measured gas concentration data against a look up table stored in memory.

## Patentansprüche

1. Verfahren (200) zum Bestimmen von Zielkomponenten in einem Probengasgemisch, umfassend:
Erhalten (210) gemessener Gaskonzentrationsdaten für jedes von einem oder mehreren in dem Probengasgemisch vorhandenen Hintergrundgasen von einem Konzentrationsmessgerät (110), wobei das Gaskonzentrationsmessgerät dazu ausgelegt ist, Gaskonzentrationen des einen oder der mehreren Hintergrundgase zu messen;
Erhalten (220) gemessener Lichtabsorptionsdaten von jeder der einen oder mehreren gasförmigen Zielkomponenten von einem Laserspektroskopiegerät, die einen Absorptionsbetrag von Licht durch das Probengasgemisch bei einer Frequenz jeder der einen oder mehreren gasförmigen Zielkomponenten anzeigen, wobei das Laserspektroskopiegerät dazu ausgelegt ist, die Lichtabsorption der einen oder mehreren gasförmigen Zielkomponenten in dem Probengas zu messen, wobei jede der einen oder mehreren gasförmigen Zielkomponenten ein Absorptionsspektrum bei der Lichtfrequenz oder bei einer Kombination von Lichtfrequenzen aufweist;
Einstellen (230) von Gaskonzentrations-Anpassungskoeffizienten für ein chemometrisches Modell für die gemessenen Gaskonzentrationsdaten, die für jedes des einen oder der mehreren in dem Probengasgemisch vorhandenen Hintergrundgase erhalten werden, wobei das chemometrische Modell eine Breitband-Ausgleichsbasis, die Absorptionen durch das eine oder die mehreren Hintergrundgase berücksichtigt, in einem End-Basissatzspektrum für die eine oder die mehreren gasförmigen Zielkomponenten einsetzt;
Anwenden (240) eines iterativen mathematischen Modells auf die gemessenen Lichtabsorptionsdaten durch iteratives Anpassen von Zielkomponenten-Anpassungskoeffizienten, bis die gemessenen Lichtabsorptionsdaten mit dem chemometrischen Modell übereinstimmen; und
Bestimmen (250) einer berechneten Menge der einen oder mehreren Zielkomponenten in dem Probengasgemisch durch Anwenden der Gaskonzentrations-Anpassungskoeffizienten und der Zielkomponenten-Anpassungskoeffizienten auf die gemessenen Lichtabsorptionsdaten und auf die gemessenen Gaskonzentrationsdaten des einen oder der mehreren Hintergrundgase.

2. Verfahren nach Anspruch 1, wobei das iterative mathematische Modell ein Regressionsmodell ist.

3. Verfahren nach Anspruch 1 oder 2, wobei ein Prozessor (120) kommunikativ mit dem Laserspektroskopiegerät und dem Gaskonzentrationsmessgerät verbunden ist, wobei der Prozessor angewiesen ist zum:
Einstellen von Gaskonzentrations-Anpassungskoeffizienten für das chemometrische Modell für die gemessenen Gaskonzentrationsdaten für jeden von einem oder den mehreren Hintergründen;
Anwenden des iterativen mathematischen Modells auf die gemessenen Lichtabsorptionsdaten durch iteratives Anpassen von Zielkomponenten-Anpassungskoeffizienten, bis die gemessenen Lichtabsorptionsdaten mit dem chemometrischen Modell übereinstimmen; und
Bestimmen der berechneten Menge der einen oder mehreren Zielkomponenten in dem Probengasgemisch durch Anwenden der Gaskonzentrations-Anpassungskoeffizienten und der Zielkomponenten-Anpassungskoeffizienten auf die gemessenen Lichtabsorptionsdaten und auf die gemessenen Gaskonzentrationsdaten.

4. Verfahren nach Anspruch 3, wobei die Messabsorptionsdaten jeder der einen oder mehreren Zielkomponenten angepasst werden durch Anweisen des Prozessors, eine iterative Korrekturfunktion auf die gemessenen Absorptionsdaten jeder der einen oder mehreren Zielkomponenten gegen eine Modelllichtabsorption der einen oder mehreren Zielkomponenten in der Standardgaskonzentration des einen oder der mehreren Hintergrundgase anzuwenden.

5. Verfahren nach einem der vorstehenden Ansprüche, wobei das Laserspektroskopiegerät eine Spektroskopievorrichtung mit außeraxial integriertem Hohlraumausgang (10) ist.

6. Verfahren nach einem der vorstehenden Ansprüche, wobei das Gaskonzentrationsmessgerät ausgewählt ist aus einer Gruppe abestehend aus einem Gaschromatographen, einer Fourier-Transformations-Infrarotspektroskopievorrichtung, einem Gaskonzentrationssensor, einem Metalloxidgassensor, einem elektrochemischen Gassensor, einem dynamischen Zirkondioxidsensor und einem katalytischen Gassensor.

7. Verfahren nach einem der vorstehenden Ansprüche, wobei die eine oder mehreren gasförmigen Zielkomponenten gasförmige Verunreinigungen sind, die in Erdgas gefunden werden.

8. Verfahren nach einem der vorstehenden Ansprüche, wobei die gasförmigen Verunreinigungen eines oder mehrere von H₂S, H₂O, O₂ oder CO₂ sind.

9. Verfahren (300) zum Bestimmen von Zielkomponenten in einem Probengasgemisch, umfassend:
Erhalten (310) gemessener Gaskonzentrationsdaten für jedes von einem oder mehreren in dem Probengasgemisch vorhandenen Hintergrundgasen von einem Konzentrationsmessgerät (110), wobei das Gaskonzentrationsmessgerät dazu ausgelegt ist, Gaskonzentrationen des einen oder der mehreren Hintergrundgase zu messen;
Erhalten (320) gemessener Lichtabsorptionsdaten von jeder der einen oder mehreren gasförmigen Zielkomponenten von einem Laserspektroskopiegerät, die einen Absorptionsbetrag von Licht durch das Probengasgemisch bei einer Frequenz jeder der einen oder mehreren gasförmigen Zielkomponenten anzeigen, wobei das Laserspektroskopiegerät dazu ausgelegt ist, die Lichtabsorption der einen oder mehreren gasförmigen Zielkomponenten in dem Probengas zu messen, wobei jede der einen oder mehreren gasförmigen Zielkomponenten ein Absorptionsspektrum bei der Lichtfrequenz oder bei einer Kombination von Lichtfrequenzen aufweist;
Anwenden (330) eines iterativen mathematischen Modells auf die gemessenen Lichtabsorptionsdaten durch iteratives Anpassen von Gaskonzentrations-Anpassungskoeffizienten und Zielkomponenten-Anpassungskoeffizienten, bis die gemessenen Lichtabsorptionsdaten mit dem chemometrischen Modell übereinstimmen, wobei das chemometrische Modell eine Breitband-Ausgleichsbasis, die Absorptionen durch das eine oder die mehreren Hintergrundgase berücksichtigt, in einem End-Basissatzspektrum für die eine oder die mehreren gasförmigen Zielkomponenten einsetzt;
Anwenden (340) einer Korrekturfunktion auf das iterative mathematische Modell, wobei Eingabeparameter der Korrekturfunktion die gemessenen Gaskonzentrationsdaten und eines oder mehrere der iterativen Gaskonzentrations-Anpassungskoeffizienten und Zielkomponenten-Anpassungskoeffizienten aus dem iterativen mathematischen Modell sind; und
Bestimmen (350) einer berechneten Menge der einen oder mehreren Zielkomponenten in dem Probengasgemisch durch Anwenden der iterierten Anpassungskoeffizienten auf die gemessenen Lichtabsorptionsdaten und auf die gemessenen Gaskonzentrationsdaten des einen oder der mehreren Hintergrundgase.

10. Verfahren nach Anspruch 9, wobei Anwenden der Korrekturfunktion Vergleichen der gemessenen Gaskonzentrationsdaten mit einer in dem Speicher gespeicherten Nachschlagetabelle umfasst.

## Revendications

1. Procédé (200) de détermination de composants cibles dans un mélange gazeux d'échantillon comprenant :
obtention (210) de données de concentration de gaz mesurée pour chacun d'un ou plusieurs gaz de fond présents dans le mélange gazeux d'échantillon à partir d'un instrument de mesure de concentration (110), l'instrument de mesure de concentration de gaz étant configuré pour mesurer les concentrations de gaz d'un ou plusieurs gaz de fond ;
obtention (220) de données d'absorption de lumière mesurée de chacun d'un ou de plusieurs composants cibles gazeux à partir d'un instrument de spectroscopie laser indiquant une quantité d'absorption de lumière par le mélange gazeux d'échantillon à une fréquence de chacun des un ou plusieurs composants cibles gazeux, l'instrument de spectroscopie laser étant configuré pour mesurer l'absorption de lumière du ou des composants cibles gazeux dans le gaz d'échantillon, chacun des un ou plusieurs composants cibles gazeux ayant un spectre d'absorption à la fréquence de la lumière ou à une combinaison de fréquences de fréquences de la lumière ;
réglage (230) de coefficients d'ajustement de concentration de gaz sur un modèle chimiométrique pour les données de concentration de gaz mesurée obtenues pour chacun des un ou plusieurs gaz de fond présents dans le mélange gazeux d'échantillon, le modèle chimiométrique utilisant une base de décalage large bande, tenant compte d'absorptions par le ou les gaz de fond, dans un spectre d'ensemble de base final défini pour le ou les composants cibles gazeux ;
application (240) d'un modèle mathématique itératif aux données d'absorption de lumière mesurée par ajustement itératif de coefficients d'ajustement de composant cible jusqu'à ce que les données d'absorption de lumière mesurée correspondent au modèle chimiométrique ; et
détermination (250) d'une quantité calculée du ou des composants cibles dans le mélange de gaz d'échantillon en appliquant les coefficients d'ajustement de concentration de gaz et les coefficients d'ajustement de composant cible aux données d'absorption de lumière mesurée et aux données de concentration de gaz mesurée du ou des gaz de fond.

2. Procédé selon la revendication 1, dans lequel le modèle mathématique itératif est un modèle de régression.

3. Procédé selon la revendication 1 ou 2, dans lequel un processeur (120) est connecté en communication à l'instrument de spectroscopie laser et à l'instrument de mesure de concentration de gaz, le processeur étant chargé de :
fixer des coefficients d'ajustement de la concentration de gaz au modèle chimiométrique pour les données de concentration de gaz mesurée pour chacun d'un ou de plusieurs fonds ;
appliquer le modèle mathématique itératif aux données d'absorption de lumière mesurée en ajustant itérativement des coefficients d'ajustement de composant cible jusqu'à ce que les données d'absorption de lumière mesurée correspondent au modèle chimiométrique ; et
déterminer la quantité calculée d'un ou plusieurs composants cibles du ou des mélanges de gaz d'échantillon en appliquant les coefficients d'ajustement de concentration de gaz et les coefficients d'ajustement de composant cible aux données d'absorption de lumière mesurée et aux données de concentration de gaz mesurée.

4. Procédé selon la revendication 3, dans lequel les données d'absorption de mesure de chacun des un ou plusieurs composants cibles sont ajustées en ordonnant au processeur d'appliquer une fonction de correction itérative aux données d'absorption mesurée de chacun des un ou plusieurs composants cibles par rapport à une absorption de lumière modèle des un ou plusieurs composants cibles dans la concentration de gaz standard du ou des gaz de fond.

5. Procédé selon l'une quelconque des revendications précédentes, dans lequel l'instrument de spectroscopie laser est un dispositif de spectroscopie intégrée avec sortie de cavité hors-axe (10).

6. Procédé selon l'une quelconque des revendications précédentes, dans lequel l'instrument de mesure de concentration de gaz est choisi dans un groupe constitué d'un chromatographe en phase gazeuse, d'un dispositif de spectroscopie infrarouge à transformée de Fourier, d'un capteur de concentration de gaz, d'un capteur de gaz à oxyde métallique, d'un capteur de gaz électrochimique, d'un capteur de dioxyde de zircone dynamique et d'un capteur de gaz catalytique.

7. Procédé selon l'une quelconque des revendications précédentes, dans lequel le ou les composants cibles gazeux sont des contaminants gazeux trouvés dans le gaz naturel.

8. Procédé selon l'une quelconque des revendications précédentes, dans lequel les contaminants gazeux sont un ou plusieurs parmi H₂S, H₂O, O₂ ou CO₂.

9. Procédé (300) de détermination de composants cibles dans un mélange gazeux d'échantillon comprenant :
obtention (310) de données de concentration de gaz mesurée pour chacun d'un ou plusieurs gaz de fond présents dans le mélange gazeux d'échantillon à partir d'un instrument de mesure de concentration (110), l'instrument de mesure de concentration de gaz étant configuré pour mesurer les concentrations de gaz d'un ou plusieurs gaz de fond ;
obtention (320) de données d'absorption de lumière mesurée de chacun d'un ou de plusieurs composants cibles gazeux à partir d'un instrument de spectroscopie laser indiquant une quantité d'absorption de lumière par le mélange gazeux d'échantillon à une fréquence de chacun des un ou plusieurs composants cibles gazeux, l'instrument de spectroscopie laser étant configuré pour mesurer l'absorption de lumière du ou des composants cibles gazeux dans le gaz d'échantillon, chacun des un ou plusieurs composants cibles gazeux ayant un spectre d'absorption à la fréquence de la lumière ou à une combinaison de fréquences de fréquences de la lumière ;
application (330) d'un modèle mathématique itératif aux données d'absorption de lumière mesurée en ajustant itérativement des coefficients d'ajustement de concentration de gaz et des coefficients d'ajustement de composant cible jusqu'à ce que les données d'absorption de lumière mesurée correspondent au modèle chimiométrique, le modèle chimiométrique utilisant une base de décalage large bande, tenant compte d'absorptions par le ou les gaz de fond, dans un spectre de base final défini pour le ou les composants cibles gazeux ;
application (340) d'une fonction de correction au modèle mathématique itératif, dans lequel les paramètres d'entrée de la fonction de correction sont les données de concentration de gaz mesurée et un ou plusieurs des coefficients d'ajustement de la concentration de gaz itérés et des coefficients d'ajustement de composant cible du modèle mathématique itératif ; et
détermination (350) d'une quantité calculée du ou des composants cibles dans le mélange de gaz d'échantillon en appliquant les coefficients d'ajustement itérés aux données d'absorption de lumière mesurée et aux données de concentration de gaz mesurée du ou des gaz de fond.

10. Procédé selon la revendication 9, dans lequel l'application de la fonction de correction comprend la comparaison des données de concentration de gaz mesurée avec une table de consultation stockée dans une mémoire.
